# EUROPÄISCHE PATENTSCHRIFT

(11) **EP 3 049 143 B1**
(45) Veröffentlichungstag und Bekanntmachung des Hinweises auf die Patenterteilung: **10.07.2019**
(21) Anmeldenummer: 14789504.9
(22) Anmeldetag: 23.09.2014
(51) Int. Cl.: A61M 25/02, A61M 25/01, A61M 27/00

(54) **PLATZIERVORRICHTUNG FÜR EINEN MEDIZINISCHEN KATHETER**
POSITIONING DEVICE FOR A MEDICAL CATHETER
DISPOSITIF DE POSE POUR UN CATHÉTER MÉDICAL

(30) Priorität: 24.09.2013 DE 102013015845
(43) Veröffentlichungstag der Anmeldung: 03.08.2016
(73) Patentinhaber: Urotech GmbH, 83101 Rohrdorf-Achenmühle (DE)
(72) Erfinder: SCHWARZ, Werner, 83324 Ruhpolding (DE); HAUSER, David, 83022 Rosenheim (DE); JANSSEN, Christopher, 27321 Thedinghausen-Wulmstorf (DE)
(74) Vertreter: Hofstetter, Schurack & Partner
(86) Internationale Anmeldenummer: PCT/DE2014/000481
(87) Internationale Veröffentlichungsnummer: WO 2015/043569

(56) Entgegenhaltungen:
- EP-A2- 2 371 308
- EP-A2- 2 604 231
- EP-B1- 1 459 780
- WO-A1-2007/029242
- DE-T2- 69 831 342

## Beschreibung

Die vorliegende Erfindung bezieht sich auf eine Platziervorrichtung für einen in einer Körperöffnung oder -höhle eines Individuums zu platzierenden medizinischen Katheter, umfassend ein längliches rohr- oder schlauchförmiges Katheter-Aufnahmeglied, von dessen einen Ende der zu platzierende Katheter aufnehmbar ist, ein innerhalb oder außerhalb des Katheter-Aufnahmegliedes aufgenommenes und in dessen Längsrichtung verschiebbares längliches Katheter-Löseglied und einen zwischen dem Katheter-Aufnahmeglied und dem Katheter-Löseglied vorgesehenen Bewegungsmechanismus mit einer Bewegungsumsetzeinrichtung, durch die das Katheter-Aufnahmeglied mit dem von diesem aufgenommenen Katheter und das Katheter-Löseglied in einer Vorrichtungslängsrichtung relativ zueinander unter Loslösen des Katheters von dem Katheter-Aufnahmeglied verschiebbar sind.

Eine Platziervorrichtung der vorstehend genannten Art ist bereits bekannt (EP 1 459 780 B1). Diese bekannte Platziervorrichtung besteht aus einer an einem Ende des Katheters lösbar mit diesem verbindbaren länglich ausgebildeten Haltevorrichtung, die von einem Schubschlauch derart umgeben ist, dass im Gebrauch ein distales Ende des Schubschlauches im Bereich des Endes des Katheters zu liegen kommt. Der Schubschlauch ist dabei mittels eines beweglichen Schubelements koaxial zu der Haltevorrichtung derart verschiebbar, dass bei einer Verschiebung des Schubschlauches in distaler Richtung das Ende des Katheters von dem distalen Ende der Haltevorrichtung lösbar ist und das Schubelement, die Haltevorrichtung und der Schubschlauch zumindest teilweise innerhalb des Gehäuses angeordnet sind, welches mindestens eine distale Öffnung für den Austritt der Haltevorrichtung und/oder des Schubschlauches aufweist.

Das Gehäuse besteht dabei aus einem Gehäuseoberteil und einem Gehäuseunterteil. Das Gehäuseoberteil und das Gehäuseunterteil sind zueinander drehbar ausgebildet, und an einem Innenumfang des Gehäuseoberteils ist zumindest eine Führung zur Führung von zumindest einem an dem Schubelement ausgebildeten Rastelement angeordnet. Die Führung weist zumindest zwei Rastausnehmungen zur Aufnahme des Rastelements auf, und die Rastausnehmungen sind in distaler Richtung zueinander versetzt ausgebildet. Durch Ausführen einer Drehbewegung zwisehen dem Gehäuseoberteil und dem Gehäuseunterteil ermöglicht es die betreffende bekannte Platziervorrichtung, dem Schubschlauch eine Longitudinalbewegung zu erteilen, durch die der Katheter vom distalen Ende der Haltevorrichtung lösbar ist. Die Ausführung der genannten Drehbewegung erfordert indessen normalerweise eine Zweihandbedienung der betreffenden bekannten Platziervorrichtung.

Es ist auch schon eine Vorrichtung zum Implantieren eines durch eine Ureterschiene gebildeten Katheters in einem Harnleiter bekannt (DE 201 13 815 U1). Dieses bekannte Applikationsbesteck umfasst eine Außenkanüle, ein Kopfstück, das mit dem einen Ende der Außenkanüle fest verbunden ist, eine Griffhülse, die mit dem Kopfstück lösbar verbunden ist, und einen Längsschlitz, welcher zum einen Ende der Griffhülse hin offen ist. Ferner ist eine Innenkanüle vorgesehen, die in der Außenkanüle axial verschiebbar angeordnet ist und die sich durch die Griffhülse hindurch erstreckt. Außerdem ragt ein von einer vorzugsweise als Druckfeder ausgebildeten Feder in Richtung des Kopfstücks gedrücktes Betätigungselement, das mit der Innenkanüle fest verbunden ist, durch den Längsschlitz der Griffhülse hindurch und wird in diesem geführt. Überdies ist ein Endstück mit der Griffhülse lösbar verbunden. Ein Applizieren einer Ureterschiene in einem Harnleiter erfordert allerdings auch hier normalerweise eine Zweihandbedienung des betreffenden bekannten Applikationsbestecks.

Aus EP 2 371 308 A2 ist ein medizinisches Instrument bekannt, das einen hohlen Schaft aufweist, an dessen proximalen Ende eine aus mindestens zwei Griffteilen bestehende Handhabe und an dessen distalen Ende ein Werkzeug angeordnet sind. Das Werkzeug ist mittels der Handhabe über ein in dem Schaft axial verschiebbar gelagertes Betätigungselement zu betätigen, welches ein Zug-/Druckelement aufweist, das als axialverschiebbar im Schaft gelagertes kreuzförmiges Element mit einem in Instrumentenlängsachse ausgerichteten Grundkörper und wenigstens zwei nach außen abstehenden, stabförmigen Armen ausgebildet ist. Die Griffteile der Handhabe und die Arme des Zug-/ Druckelements stehen über eine Zapfen-Schlitz-Steuerung in Wirkverbindung miteinander.

Zuweilen besteht jedoch der Wunsch, Platziervorrichtungen für in Körperöffnungen oder-höhlen von Individuen zu platzierenden medizinischen Kathetern jeweils mit nur einer Hand bedienen zu können. Der Erfindung liegt daher die Aufgabe zu Grunde eine Platziervorrichtung der eingangs genannten Art so auszubilden, dass sie eine Einhandbedienung ermöglicht.

Gelöst wird die vorstehend genannte Aufgabe durch eine Platziervorrichtung mit den im Patentanspruch 1 angegebenen Merkmalen.

Hierdurch ergibt sich der Vorteil, dass mit relativ geringem Aufwand eine Einhandbedienung der Platziervorrichtung ermöglicht ist. Nach Aufnehmen der erfindungsgemäßen Platziervorrichtung in der Handfläche einer Bedienungshand einer Bedienungsperson kann dann das Betätigungsglied beispielsweise einfach mit dem Daumen der betreffenden Hand betätigt werden, um den Katheter nach Platzieren an gewünschter Stelle von dem Katheter-Aufnahmeglied zu lösen.

Bei gesonderter Ausbildung der Drückteile und des Verbindungsteiles weisen die Gelenkglieder vorzugsweise einen mit dem Katheter-Löseglied oder dem Katheter-Aufnahmeglied fest verbundenen Tragteil und von diesem abstehende Tragarme auf, die mit Koppelelementen des Betätigungsgliedes gelenkig verbunden sind. Dadurch lässt sich auf besonders einfache Weise zwischen den beiden Drückteilen und dem Verbindungsteil eine Gelenkanordnung realisieren, die problemlos für eine Vielzahl von wiederholten Gelenkbewegungen geeignet ist. Eine derart aufgebaute Platziervorrichtung ist dann ohne weiteres für wiederholte Anwendungen nutzbar.

Vorzugsweise können die Gelenkglieder an den beiden Drückteilen und dem Verbindungsteil so angeformt sein, dass sämtliche dieser Elemente durch ein einziges zusammenhängendes Teil gebildet sind.

Gemäß einer noch weiteren zweckmäßigen Ausgestaltung der Erfindung ist zwischen dem Katheter-Aufnahmeglied und dem Katheter-Löseglied eine strahlungsempfindliche Detektierschicht vorgesehen. Dies bringt den Vorteil mit sich, dass das distale Ende der Platziervorrichtung gemäß der Erfindung, von welchem der in einem Individuum zu platzierende Katheter getragen wird, durch ein bildgebendes Verfahren auf einem Monitor sichtbar gemacht werden kann. Vorzugsweise wird hier die Strahlungsempfindlichkeit für Röntgenstrahlung ausgenutzt. Dazu besteht die betreffende strahlungsempfindliche Detektierschicht aus Gold, Platin oder aus einer Platin-Iridium-Legierung, die entweder auf die Außenseite des Katheter-Aufnahmegliedes oder auf die Innenseite des Katheter-Lösegliedes aufgebracht ist.

An Hand von Zeichnungen wird die Erfindung nachstehend an einem konkreten Ausführungsbeispiel einer Platziervorrichtung gemäß der Erfindung erläutert.
- Fig. 1: zeigt in einer nicht maßstäblichen Seitenansicht eine Ausführungsform einer Platziervorrichtung.
- Fig. 2: zeigt in einer nicht maßstäblichen Perspektivansicht die in Fig. 1 dargestellte Platziervorrichtung im nicht zusammengedrückten Zustand.
- Fig. 3: zeigt eine Vorderansicht der in Fig. 2 dargestellten Platziervorrichtung.
- Fig. 4: zeigt in einer nicht maßstäblichen Schnittansicht die in Fig. 1 dargestellte Platziervorrichtung im nicht zusammengedrückten Zustand.
- Fig. 5: zeigt in einer nicht maßstäblichen Schnittansicht die in Fig. 1 dargestellte Platziervorrichtung in einem zusammengedrückten Zustand.
- Fig. 6: zeigt in einer nicht maßstäblichen Seitenansicht ein Ausführungsbeispiel einer Platziervorrichtung gemäß der Erfindung im nicht zusammengedrückten Zustand.
- Fig. 7: zeigt in einer Vorderansicht die in Fig. 6 dargestellte Platziervorrichtung.
- Fig. 8: zeigt in einer nicht maßstäblichen Schnittansicht die in Fig. 6 dargestellte Platziervorrichtung im nicht zusammengedrückten Zustand.
- Fig. 9: zeigt in einer nicht maßstäblichen Schnittansicht die in Fig. 6 dargestellte Platziervorrichtung in einem zusammengedrückten Zustand.
- Fig. 10: zeigt in einer nicht maßstäblichen und zum Teil geschnittenen Perspektivansicht eine weitere Ausführungsform einer Platziervorrichtung im nicht betätigten Zustand.
- Fig. 11: zeigt die in Fig. 10 dargestellte Platziervorrichtung im nicht betätigten Zustand in einer Schnittansicht.
- Fig. 12: zeigt die in Fig. 10 dargestellte Platziervorrichtung im betätigten Zustand in der Schnittansicht.
- Fig. 13: zeigt in einer nicht maßstäblichen Perspektivansicht eine noch weitere Ausführungsform einer Platziervorrichtung im nicht betätigten Zustand.
- Fig. 14: zeigt die in Fig. 13 dargestellte Platziervorrichtung im nicht betätigten Zustand in einer Schnittansicht.
- Fig. 15: zeigt die in Fig. 13 dargestellte Platziervorrichtung im betätigten Zustand in der Schnittansicht.
- Fig. 16: zeigt in einer ähnlichen nicht maßstäblichen Perspektivansicht wie Fig. 2 eine noch weitere Ausführungsform einer Platziervorrichtung im nicht zusammengedrückten Zustand zusammen mit einem Sicherungsteil.
- Fig. 17: zeigt die in Fig. 16 dargestellte Platziervorrichtung mit herausgenommenem Sicherungsteil in zwei verschiedenen Stellungen.
- Fig. 18: zeigt in einer nicht maßstäblichen Schnittansicht den Bereich zwischen einem Katheter-Aufnahmeglied und einem Katheter-Löseglied einer Platziervorrichtung gemäß der Erfindung.

Bevor auf die Zeichnungen näher eingegangen wird, sei zunächst angemerkt, dass entsprechende Teile bzw. Elemente in sämtlichen Zeichnungsfiguren durch gleiche Bezugszeichen bezeichnet sind.

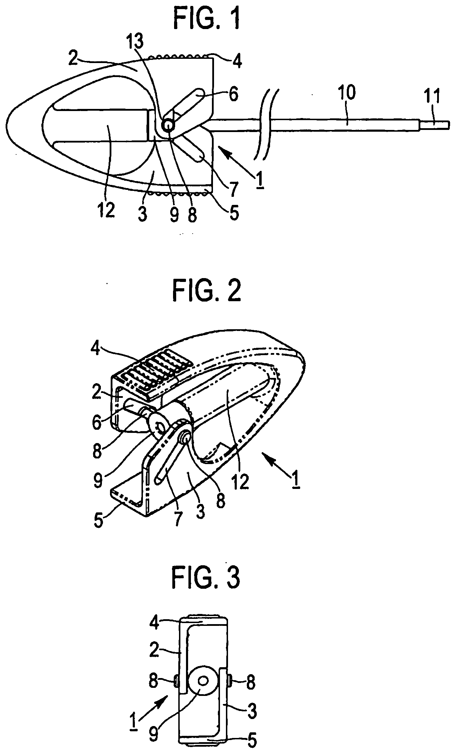

Fig. 1 zeigt in einer Seitenansicht eine erste Ausführungsform einer Platziervorrichtung 1 in einer Seitenansicht. Die Platziervorrichtung 1 besteht aus einem zweiteiligen Betätigungskörper, der - wie dies aus Fig. 2 und 3 näher ersichtlich ist - zwei parallel in relativ geringem Abstand voneinander verlaufende Seitenteile 2 und 3 aufweist, die an ihren einen Enden miteinander verbunden sind - das sind ihre in Fig. 1 links dargestellten Enden. Von den beiden Seitenteilen 2, 3 stehen hier rechtwinklig zu ihnen verlaufende Drückteile 4 bzw. 5 ab, die in Richtung zu dem jeweils anderen Seitenteil 3 bzw. 2 verlaufen.

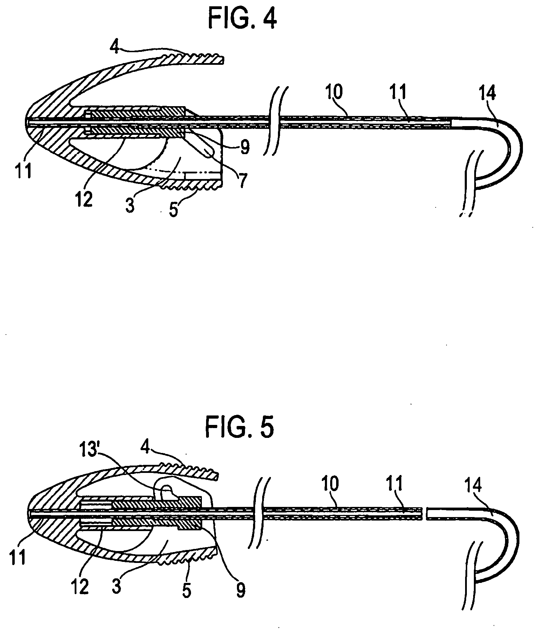

Von dem in Fig. 1 nicht näher bezeichneten Verbindungsbereich der einen erwähnten Enden der Seitenteile 2, 3 steht ein in den Zwischenraum zwischen den beiden Drückteilen 4, 5 hinein ragender Tragteil 12 hinein, von welchem ein Antriebselement 9 verschiebbar aufgenommen ist, wie dies aus den Schnittansichten gemäß Fig. 4 und 5 näher ersichtlich ist. Dieses Antriebselement 9 ist hier durch ein zylindrisches Teil gebildet, von dem quer zur Vorrichtungslängsrichtung zumindest ein Lagerzapfen 8 absteht, der in einer Führungsbahn 6 bzw. 7 geführt ist, die sich in dem dem betreffenden Lagerzapfen zugewandten Seitenteil 2 bzw. 3 befindet. Im vorliegenden Fall stehen den von dem Antriebselement 9 zwei derartige Lagerzapfen 8 von diametral gegenüber liegenden Stellen ab. Diese Lagerzapfen 8 sind in Führungsbahnen 6, 7 geführt, die in Bezug auf die Vorrichtungslängsrichtung der Platziervorrichtung 1 in entgegen gesetzte Richtungen verlaufen, wie dies aus Fig. 1 ersichtlich ist. In der Führungsbahn 6 ist noch eine Führungsbahnabsenkunkung 13 gezeigt, auf deren Bedeutung weiter unten noch näher eingegangen wird.

Die Platziervorrichtung 1 umfasst ferner ein Katheter-Aufnahmeglied 11, welches hier von einem Katheter-Löselied 10 aufgenommen ist. Auf diese beiden Glieder 11 und 10 wird weiter unten noch näher eingegangen.

Es sei an dieser Stelle angemerkt, dass die Führungsbahnen 6, 7 gegebenenfalls durch einfache Führungsflächen ersetzt sein können, die in gleichen Richtungen verlaufen wie die Führungsbahnen 6, 7. Unter der erwähnten Vorrichtungslängsrichtung ist übrigens im Rahmen dieser Anmeldung die Längsrichtung der jeweiligen Platziervorrichtung und damit diejenige Richtung zu verstehen, in der das Katheter-Aufnahmeglied 11 und das Katheter-Löseglied 10 verlaufen.

Der vorstehend an Hand der Fig. 1, 2 und 3 erläuterte Aufbau der Platziervorrichtung 1 mit den Seitenteilen 2, 3, den Drückteilen 4, 5, den Führungsbahnen 6, 7, den Lagerzapfen 8, dem Antriebselement 9 und dem Tragteil 12 stellt - wie dies aus den Darstellungen gemäß Fig. 4 und 5 näher ersichtlich ist - eine Bewegungsumsetzeinrichtung dar, in der die Drückteile 4, 5 eine Betätigungseinrichtung darstellen, durch deren Betätigen in einer quer zur Vorrichtungslängsrichtung erfolgenden Richtung das Antriebselement 9 in der genannten Vorrichtungslängsrichtung relativ zu dem Tragteil 12 verschoben wird. Dies heißt, dass durch Betätigen, nämlich durch Zusammendrücken der beiden Drückteile 4, 5 eine dieser Bewegung entsprechende Transversalbewegung in eine Longitudinalbewegung des Antriebselements 9 relativ zu dem Tragteil 12 umsetzbar ist.

Mit dem zuvor erwähnten Tragteil 12 ist, wie aus Fig. 4 und 5 näher ersichtlich wird, das schlauch- bzw. zylinderförmige Katheter-Aufnahmeglied 11 fest verbunden. Dieses Katheter-Aufnahmeglied 11 dient zur lösbaren Aufnahme des zu platzierenden Katheters 14 (in Fig. 1 nicht dargestellt), der dazu auf das betreffende Katheter-Aufnahmeglied 11 aufgesteckt wird. Das Katheter-Aufnahmeglied 11 ist gemäß Fig. 1 von dem länglichen Katheter-Löseglied 10 umgeben, bei dem es sich vorzugsweise um ein schlauchförmiges Katheter-Löseglied 10 handelt. Dieses Katheter-Löseglied 10 ist bei der vorliegenden Ausführungsform mit dem Antriebselement 9 fest verbunden, welches in der Vorrichtungslängsrichtung relativ zu dem Tragteil 12 verschiebbar ist.

Die vorstehend erwähnte Umsetzung einer Transversalbewegung in eine Longitudinalbewegung geht aus den in Fig. 4 und 5 gezeigten Schnittdarstellungen der zuvor an Hand der Fig: 1, 2 und 3 erläuterten ersten Ausführungsform einer Platziervorrichtung 1 klar hervor.

Dabei zeigt Fig. 4 den aus Fig. 1 ersichtlichen Zustand der betreffenden Platziervorrichtung 1. In diesem Zustand befinden sich die beiden Drückteile 4 und 5 im nicht zusammengedrückten Ausgangszustand. Der Katheter 14, bei dem es sich beispielsweise um eine Ureterschiene handeln kann, ist auf das in Fig. 4 rechts dargestellte distale Vorrichtungsende auf das Katheter-Aufnahmeglied 10 aufgesteckt, beispielsweise mittels einer in ihm enthaltenen Längsöffnung.

Fig. 5 zeigt die betreffende Platziervorrichtung in einem Zustand, in welchem die beiden Drückteile 4 und 5 zusammengedrückt sind. Ein Vergleich dieser Darstellung mit der in Fig. 4 gezeigten Darstellung lässt erkennen, dass durch das Zusammendrücken und der damit erfolgten Ausübung einer Transversalbewegung in Bezug auf die Vorrichtungslängsrichtung der beiden Drückteile 4 und 5 das Antriebselement 9 durch eine Longitudinalbewegung aus dem Tragteil 12 heraus geschoben worden ist. Durch dieses Herausschieben ist das Katheter-Löseglied 10 relativ zu dem Katheter-Aufnahmeglied 11 soweit zu dem distalen Vorrichtungsende hin verschoben worden, dass es im vorliegenden Fall mit dem distalen Ende des Katheter-Aufnahmegliedes 11 bündig abschließt oder sogar über dieses Ende hinausragt. Durch diese Longitudinalbewegung ist somit der Katheter 14 von dem Katheter-Aufnahmeglied 11 gelöst.

Der zuvor erläuterte Vorgang des Lösens des Katheters 14 von dem Antriebselement 9 wird üblicherweise erst nach erfolgtem Platzieren des Katheters 14 mittels der beschriebenen Platziervorrichtung 1 in einer Körperhöhle oder -öffnung eines Individuums vorgenommen. Um ein unbeabsichtigtes Lösen des Katheters 14 von dem Katheter-Aufnahmeglied 11 während des Anbringens des Katheters 14 an diesem Katheter-Aufnahmeglied 11 zu vermeiden, weisen die Führungsbahnen 6, 7 an den Stellen, an denen diese die Lagerzapfen 8 in der nicht betätigten Ausgangsstellung der Drückteile 4, 5 aufnehmen, jeweils eine Führungsbahnabsenkung 13 bzw. 13' auf, die praktisch rechtwinklig zu der Vorrichtungslängsrichtung verlaufen. Wird bei in diesen Führungsbahnabsenkungen 13 bzw. 13' befindlichen Lagerzapfen 8 ein Druck durch in Fig. 1, 4 und 5 nach links - also zum proximalen Vorrichtungsende hin durch das Katheter-Aufnähmeglied 11 ausgeübt, so führt dieser Druck lediglich dazu. Dass die Lagerzapfen 8 gegen die Führungsbahnabsenkungen 13, 13' gedrückt werden; sie können durch diesen Druck jedenfalls nicht aus den betreffenden Führungsbahnabsenkungen 13' herausgehoben werden. Damit ist in dieser Stellung ein Verschieben des Katheter-Aufnahmegliedes 11 in Bezug auf das Katheter-Löseglied 10 verhindert. Ein Herausführen der Lagerzapfen 8 aus den Führungsbahnabsenkungen 13, 13' ist lediglich durch Zusammendrücken der Drückteile 4, 5 möglich.

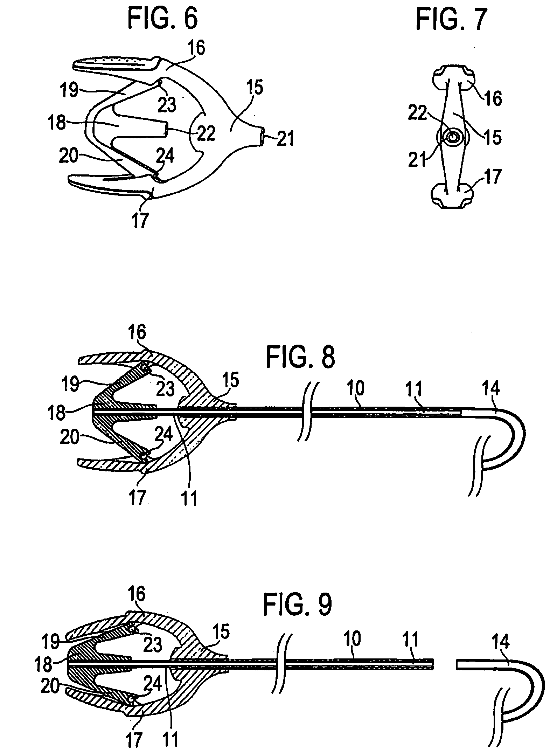

In Fig. 6, 7, 8 und 9 ist ein Ausführungsbeispiel der Platziervorrichtung gemäß der Erfindung veranschaulicht. Im Unterschied zu der in den Fig. 1, 2, 3, 4 und 5 veranschaulichten Ausführungsform besteht hier die Bewegungsumsetzeinrichtung, mittels der eine Transversalbewegung in eine Longitudinalbewegung umsetzbar ist, aus zwei Drückteilen 16, 17 und einer diese Drückteile 16, 17 aufnehmenden Trageinrichtung 18, 19, 20.

Die beiden Drückteile 16, 17 sind in ihren Bereichen zur Vorrichtungsmitte hin durch ein Verbindungsteil 15 miteinander verbunden, um das sie - wie dies an Hand der Schnittansichten gemäß Fig. 8 und 9 noch ersichtlich wird - elastisch miteinander zusammendrückbar sind. Die zuvor erwähnte Trageinrichtung besteht hier aus einem zwei Tragarme 19, 20 und einem diese verbindenden Verbindungsteil 18, um den die beiden Tragarme elastisch zusammendrückbar sind.

Die Tragarme 19, 20 können prinzipiell mit den Drückteilen 16 bzw. 17 zusammenhängend einteilig ausgebildet sein, beispielsweise als ein vollständig zusammenhängendes Spritzgussteil. Im vorliegenden Fall sind jedoch zwischen den Enden der durch den Verbindungsteil 18 miteinander verbundenen Tragarme 19, 20 und inneren Anlenkstellen der Drückteile 16, 17 Gelenkglieder 23, 24 vorgesehen. Diese Gelenkglieder 23, 24 bestehen jeweils aus einer Gelenkkugel und einer diese ausnehmenden Gelenkpfanne, wie dies die Schnittansichten gemäß Fig. 8 und 9 klar erkennen lassen.

Die beiden Drückteile 16, 17 und die innerhalb dieser Drückteile 16, 17 liegenden Tragarme 18, 19 bilden - wie die Seitenansicht gemäß Fig. 7 erkennen lässt - wie die in Fig. 1 bis 5 dargestellte Platziervorrichtung 1 eine relativ schmale Platziervorrichtung. Der Verbindungsteil 15 weist dabei eine Durchgangsöffnung 21 relativ großer Öffnungsweite (Durchmesser) zur festen Aufnahme des schlauchförmigen Katheter-Lösegliedes 10 auf. Der Verbindungsteil 18 weist eine Durchgangsöffnung 22 auf, die jedoch eine gegenüber der Öffnungsweite der Durchgangsöffnung 21 geringere Öffnungsweite (Durchmesser) aufweist. In dieser Durchgangsöffnung 22 ist das für die Aufnahme eines Katheters 14 vorgesehene Katheter-Aufnahmeglied 11 fest aufgenommen, welches in dem schlauchförmigen Katheter-Löseglied verschiebbar aufgenommen ist.

Die Platziervorrichtung gemäß diesem Ausführungsbeispiel der Erfindung wird in der Weise betätigt, wie dies aus den Schnittansichten gemäß Fig. 8 und 9 ersichtlich ist. In der Schnittansicht gemäß Fig. 8 befindet sich die Platziervorrichtung in ihrem Ausgangszustand, in welchem die Drückteile 16, 17 nicht zusammengedrückt sind und in welchem ein Katheter 14 mit seinem einen Ende von dem distalen Ende des Katheter-Aufnahmegliedes 11 lösbar aufgenommen ist.

Werden sodann die beiden Drückteile 16, 17 zusammengedrückt und in den aus Fig. 9 ersichtlichen Zustand überführt, so lässt ein Vergleich dieser Darstellung mit der in Fig. 8 gezeigten Darstellung erkennen, dass auch hier durch das Zusammendrücken der beiden Drückteile 16, 17 und der damit erfolgten Ausübung einer Transversalbewegung in Bezug auf die Vorrichtungslängsrichtung der beiden Drückteile 16 und 17 eine solche relative Longitudinalbewegung zwischen dem Katheter-Aufnahmeglied 11 und dem Katheter-Löseglied 10 erfolgt, dass das distale Ende des Katheter-Lösegliedes 10 im vorliegenden Fall mit dem distalen Ende des Katheter-Aufnahmegliedes 11 bündig abschließt oder sogar über dieses Ende hinausragt. Durch diese Longitudinalbewegung ist somit der Katheter 14 von dem Katheter-Aufnahmeglied 11 gelöst.

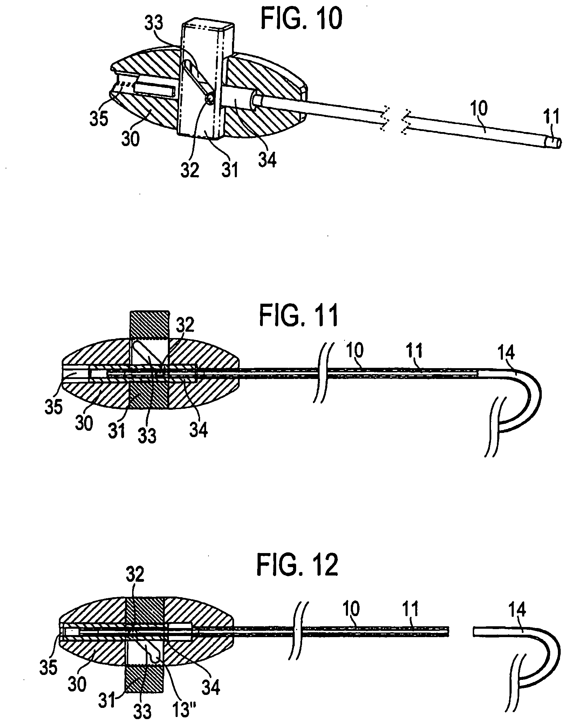

In Fig. 10, 11 und 12 ist eine weitere Ausführungsform einer Platziervorrichtung veranschaulicht. Im Unterschied zu den zuvor betrachteten Ausführungsformen besteht hier die Bewegungsumsetzeinrichtung, mittels der eine Transversalbewegung in eine Longitudinalbewegung umsetzbar ist, aus einem Halter 30, mit dem das schlauchförmige Katheter-Löseglied 10 fest verbunden ist, und einem tastenförmigen Betätigungsglied 31, welches durch Druckausübung winklig, und zwar hier rechtwinklig zur Vorrichtungslängsrichtung bewegbar ist.

Das Betätigungsglied 31 weist in zumindest einer seiner in der Zeichenebene liegenden Seiten eine schräg zur Vorrichtungslängsachse geneigte Führungsbahn 33 auf, von der ein Lagerzapfen 32 einer Zapfenanordnung 32 aufgenommen ist. Diese(r) Lagerzapfen 32 ist/sind mit einem Schiebeglied 34 fest verbunden, welches in einem Längsloch 35 in dem Halter 30 verschiebbar aufgenommen ist. Mit diesem Schiebeglied 34 ist das schlauchförmige Katheter-Aufnahmeglied 11 fest verbunden, dessen in den Fig. 10 und 11 rechts dargestelltes distales Ende zur Aufnahme eines Katheters 14 dient.

Wenn das tastenförmige Betätigungsglied 31 durch Druckausübung von dem aus Fig. 11 ersichtlichen Zustand - in welchem das Betätigungsglied 31 noch nicht herunter gedrückt ist - in den aus Fig. 12 ersichtlichen Zustand überführt ist - in welchem das Betätigungsglied 31 herunter gedrückt ist - ist das Schiebeglied 34 durch das Zusammenwirken des/der Lagerzapfen 32 und der Führungsbahn(en) 33 zum proximalen Vorrichtungsende, das heißt in Fig. 11 zur linken Seite hin verschoben. Zugleich ist mit dieser Verschiebung ein solches Zurückziehen des mit dem Schiebeglied 34 fest verbundenen schlauchförmigen Katheter-Aufnahmegliedes 11 erfolgt, dass dessen in Fig. 12 rechts liegendes distales Ende soweit in das schlauchförmige Katheter-Löseglied 10 eingezogen ist, dass der zuvor von dem distalen Ende des schlauchförmigen Katheter-Aufnahmegliedes 11 aufgenommene Katheter 14 von diesem Katheter-Aufnahmeglied 11 gelöst ist.

Auch bei dieser weiteren Ausführungsform kann die jeweilige Führungsbahn 33 eine Führungsbahnabsenkung 13" an ihrem einen Ende aufweisen, welches den zugehörigen Lagerzapfen 32 im nicht betätigten Zustand (siehe Fig. 10 und 11) des Betätigungsgliedes 31 aufnimmt. Der Zweck dieser Führungsbahnabsenkung 13" ist derselbe wie jener, der im Zusammenhang mit den Führungsbahnabsenkungen 13, 13' in den Fig. 1 bis 5 erläutert worden ist.

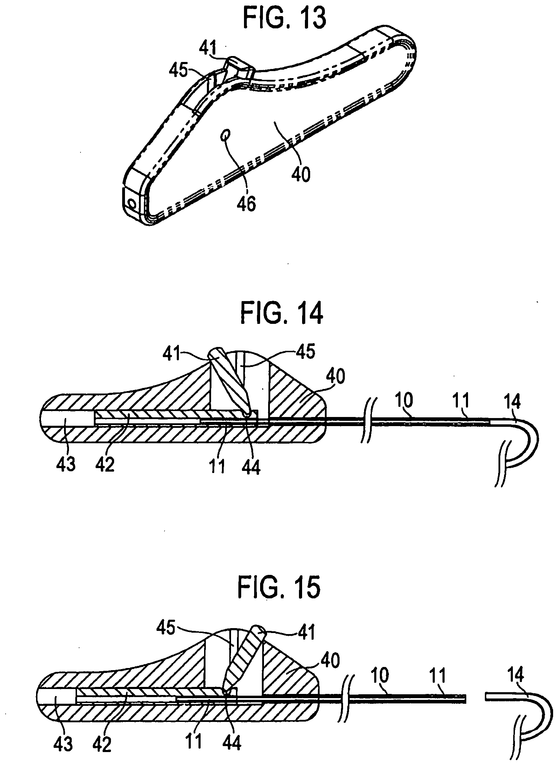

In Fig. 13, 14 und 15 ist eine noch weitere Ausführungsform der Platziervorrichtung veranschaulicht. Im Unterschied zu den zuvor betrachteten Ausführungsformen besteht hier die Bewegungsumsetzeinrichtung, mittels der eine Schwenkbewegung eines Schwenkgliedes oder Hebels 41 in eine in Vorrichtungslängsrichtung erfolgende Longitudinalbewegung umsetzbar ist, aus einem schmalen Aufnahmekörper 40, mit dem das schlauchförmige Katheter-Löseglied 10 fest verbunden ist, und dem in einer Aufnahmeöffnung 45 dieses Aufnahmekörpers 40 schwenkbar aufgenommenen Schwenkglied oder Hebel 41. Der um einen lediglich in Fig. 13 dargestellten Lagerzapfen 46 schwenkbare Hebel 41 wirkt im Innern der Aufnahmeöffnung des Aufnahmekörpers 40 auf eine Lageröffnung 44 eines Schiebegliedes 42, welches in einem in Vorrichtungslängsrichtung verlaufenden Längsloch 43 des Aufnahmekörpers 40 verschiebbar aufgenommen ist. Mit diesem Schiebeglied 42 ist das Katheter-Aufnahmeglied 11 fest verbunden, welches an seinem in Fig. 14 rechts dargestellten distalen Ende einen Katheter 14 trägt.

Durch Überführen des Schwenkgliedes bzw. Hebels 41 von der aus Fig. 14 ersichtlichen Ausgangsstellung in die aus Fig. 15 ersichtliche geschwenkte Stellung ist das Schiebeglied 42 in Fig. 15 nach links zum proximalen Vorrichtungsende hin verschoben. Zugleich mit dieser Verschiebung ist auch hier ein solches Zurückziehen des mit dem Schiebeglied 42 fest verbundenen schlauchförmigen Katheter-Aufnahmegliedes 11 erfolgt, dass dessen in Fig. 15 rechts liegendes distales Ende soweit in das schlauchförmige Katheter-Löseglied 10 eingezogen ist, dass der zuvor von dem distalen Ende des schlauchförmigen Katheter-Aufnahmegliedes 11 aufgenommene Katheter 14 von diesem Katheter-Aufnahmeglied 11 gelöst ist.

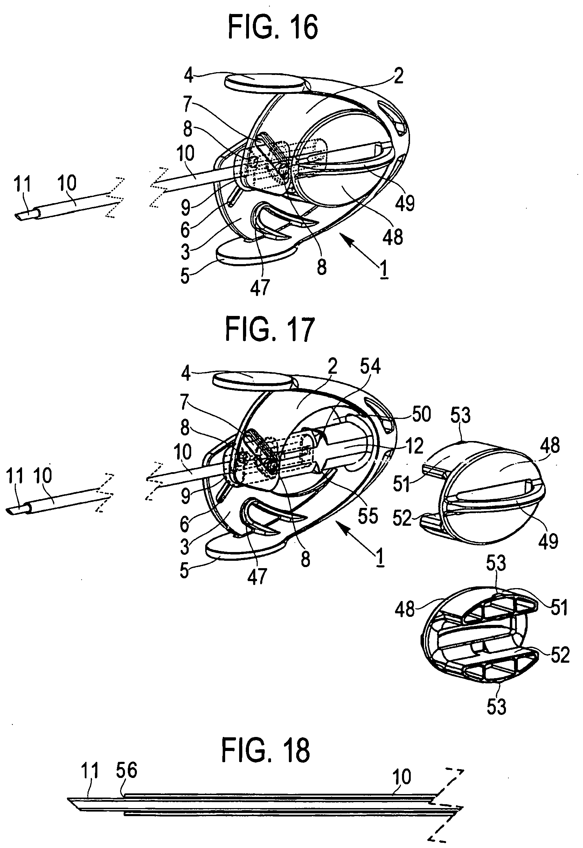

In Fig. 16 und 17 ist in einer ähnlichen nicht maßstäblichen Perspektivansicht wie Fig. 2 eine noch weitere Ausführungsform einer Platziervorrichtung 1 im nicht zusammengedrückten Zustand dargestellt. Die Platziervorrichtung 1 weist, wie aus Fig. 16 und 17 ersichtlich, ebenfalls zwei parallel in relativ geringem Abstand voneinander verlaufende Seitenteile 2 und 3 auf, die an ihren einen Enden miteinander verbunden sind - das sind ihre in Fig. 16 und 17 rechts dargestellten Enden. Von den beiden Seitenteilen 2, 3 stehen hier rechtwinklig zu ihnen verlaufende Drückteile 4 bzw. 5 ab, die in Richtung zu dem jeweils anderen Seitenteil 3 bzw. 2 verlaufen.

Von dem in Fig. 16 und 17 nicht näher bezeichneten Verbindungsbereich der einen erwähnten Enden der Seitenteile 2, 3 steht ein in einen aus Fig. 17 ersichtlichen Zwischenraum zwischen den beiden Drückteilen 4, 5 hinein ragender Tragteil 12 hinein, von welchem ein Antriebselement 9 verschiebbar aufgenommen ist, wie dies im Zusammenhang mit den Fig. 4 und 5 bereits erwähnt worden ist. Außerdem weist der Tragteil, wie aus Fig. 17 ersichtlich ist, zwei zumindest rechtwinklig in Bezug auf dessen Längsrichtung vorgesehene keilförmigen Spitzen 54 bzw. 55auf. Die Funktion dieser keilförmigen Spitzen 54 bzw. 55 wird weiter unten noch näher ersichtlich werden.

Das zuvor erwähnte Antriebselement 9 ist hier durch ein zylindrisches Teil gebildet, von dem quer zur Vorrichtungslängsrichtung zwei Lagerzapfen 8 abstehen, die in einer Führungsbahn 6 bzw. 7 geführt sind, welche sich in dem dem jeweiligen Lagerzapfen zugewandten Seitenteil 2 bzw. 3 befindet. Die beiden Lagerzapfen 8 stehen hier von diametral gegenüber liegenden Stellen ab und sind in Führungsbahnen 6, 7 geführt, die in Bezug auf die Vorrichtungslängsrichtung der Platziervorrichtung 1 in entgegen gesetzte Richtungen verlaufen, wie dies aus Fig. 16 und 17 ersichtlich ist.

Die Platziervorrichtung 1 umfasst ferner ein Katheter-Aufnahmeglied 11, welches hier von einem Katheter-Löselied 10 aufgenommen ist. Auf diese beiden Glieder 11 und 10 ist weiter oben bereits näher eingegangen worden. Aus Fig. 17 ist außerdem eine das Seitenteil 3 und dessen zugehöriges Drückteil 5 verbindende Stabilisierungsrippe 47 ersichtlich, durch die Lageposition vom Drückteil 5 in Bezug auf dessen Seitenteil 3 stabilisiert wird. Die Stabilisierungsrippe 47 wird vorzugsweise zusammen mit dem Drückteil 5 und dem Seitenteil 3 in einem einzigen Formungsvorgang hergestellt. Es sei hier noch angemerkt, dass eine der Stabilisierungsrippe 47 entsprechende Stabilisierungsrippe auch an dem Drückteil 4 und dessen Seitenteil 2 vorgesehen ist.

In Fig. 16 ist die Platziervorrichtung 1 zusammen mit einem Sicherungsteil 48 dargestellt. Dieses Sicherungsteil 48 ist in den oben in Verbindung mit Fig. 17 erwähnten Zwischenraum zwischen den beiden Drückteilen 4 lösbar eingeführt. Der betreffende Zwischenraum stellt für das Sicherungsteil 48 eine Aufnahmeöffnung 50 dar. In Fig. 17 ist gezeigt, wie das Sicherungsteil 48 aus dieser Aufnahmeöffnung 50 herausgenommen ist. Dabei ist das Sicherungsteil 48 in zwei verschiedenen Perspektivdarstellungen gezeigt. Diese beiden Perspektivdarstellungen sollen zum einen den näheren Aufbau des Sicherungsteiles 48 ersichtlich werden lassen, und zum anderen sollen sie verdeutlichen, dass das Sicherungsteil 48 von beiden Seiten der Platziervorrichtung in deren Aufnahmeöffnung 50 lösbar eingeführt werden kann, also sowohl von der aus Fig. 16 und 17 ersichtlichen Vorderseite mit dem Seitenteil 2 aus als auch von der Rückseite mit dem Seitenteil 3 aus.

Durch das in die Aufnahmeöffnung 50 der Platziervorrichtung 1 lösbar eingeführte Sicherungsteil 48 wird sichergestellt, dass ein unbeabsichtigtes Bewegen der beiden Drückteile 4, 5 durch Sperren eines Zusammendrückens verhindert werden kann. Damit steht eine besonders sicher zu handhabende Platziervorrichtung 1 zur Verfügung. Erst wenn das Sicherungsteil 48 aus der Aufnahmeöffnung 50 der Platziervorrichtung 1 herausgenommen ist, können deren Drückteile 4, 5 zusammengedrückt werden und damit die Platziervorrichtung 1 betätigt werden.

Zu dem Sicherungsteil 48 ist noch anzumerken, dass dieses einen von ihm in Bezug auf Fig. 16 und 17 nach außen abstehenden Griffteil 49 und zwei von ihm in Bezug auf Fig. 16 und 17 nach innen zu der Aufnahmeöffnung 50 hin gerichtete, hier im Querschnitt segmentförmig ausgebildete Einsteckteile 51, 52 aufweist. Zwischen diesen Einsteckteile 51, 52 ist ein durchgehender länglicher Hohlraum mit einander gegenüberliegenden Anlageflächen gebildet. Die beiden segmentförmigen Einsteckteile 51, 52 weisen jeweils aus drei miteinander verbundene Leerkammern auf. Mit den gerade erwähnten Anlageflächen liegen die beiden Einsteckteile 51, 52 des Sicherungsteiles 48 bei dessen Einführen in die Aufnahmeöffnung 50 an den oben bereits erwähnten keilförmigen Spitzen 54 bzw. 55 des Tragteiles 12 an. Diese keilförmigen Spitzen 54 bzw. 55 zentrieren gewissermaßen das Sicherungsteil 48 bei dessen Einführen in die Aufnahmeöffnung 50 der Platziervorrichtung 1.

Die beiden Einsteckteile 51, 52 sind außerdem an diametral gegenüberliegenden Stellen - hier mittig - jeweils mit einer Klemm- bzw. Rastnase 53 versehen. Mit diesen Klemm- bzw. Rastnasen 53 lässt sich das in die Aufnahmeöffnung 50 der Platziervorrichtung 1 eingeführte Sicherungsteil 48 gegen ein Herausfallen aus dieser Aufnahmeöffnung 50 sichern. Die betreffenden Klemm- bzw. Rastnasen 53 gelangen nämlich beim Einführen des Sicherungsteiles 48 in die Aufnahmeöffnung 50 der Platziervorrichtung 1 erst unter Druckausübung über jeweils einen die Aufnahmeöffnung 50 begrenzenden Randbereich der Seitenteile 2, 3 und müssen zum Herausziehen der Sicherungsteil es 48 aus der Aufnahmeöffnung 50 der Platziervorrichtung 1 unter Überwindung eines entsprechenden Druckes mit den Einsteckteilen 51, 52 aus der Aufnahmeöffnung 50 wieder herausgezogen werden.

An dieser Stelle sei noch angemerkt, dass das Sicherungsteil 48 grundsätzlich auch anders als aus Fig. 16 und 17 ersichtlich realisiert werden kann. So kann die Funktion des beschriebenen Sicherungsteil es 48 beispielsweise durch eine lösbare Verriegelungseinrichtung, wie ein Durchsteckteil durch die beiden Seitenteile 2 und 3 oder durch die beiden Drückteile 4 und 5 realisiert werden.

In Fig. 18 ist in einer nicht maßstäblichen Schnittansicht der Bereich zwischen dem Katheter-Aufnahmeglied 11 und dem Katheter-Löseglied 10 einer Platziervorrichtung 1 gemäß einer noch weiteren zweckmäßigen Ausgestaltung der Erfindung gezeigt. Hier ist zwischen dem Katheter-Aufnahmeglied 11 und dem Katheter-Löseglied 10 eine strahlungsempfindliche Detektierschicht 56 vorgesehen. Durch Bestrahlung dieses Bereiches mittels einer Strahlung, auf die die Detektierschicht 56 anspricht, lässt sich der distale Endbereich der Platziervorrichtung gemäß der Erfindung, von welchem der in einem Individuum zu platzierende Katheter getragen wird, durch ein bildgebendes Verfahren auf einem Monitor sichtbar machen. Vorzugsweise wird hier die Strahlungsempfindlichkeit für Röntgenstrahlung ausgenutzt. Dazu besteht die betreffende strahlungsempfindliche Detektierschicht aus Gold, Platin oder aus einer Platin-Iridium-Legierung, die entweder auf die Außenseite des Katheter-Aufnahmegliedes 11 oder auf die Innenseite des Katheter-Lösegliedes 10 aufgebracht ist.

Abschließend sei noch folgendes angemerkt. Bei der ersten Ausführungsform und bei dem Ausführungsbeispiel gemäß der Erfindung wurde der Katheter jeweils dadurch von seinem Katheter-Aufnahmeglied gelöst, dass das zugehörige Katheter-Löseglied über das Katheter-Aufnahmeglied geschoben wurde. Demgegenüber wurde der Katheter bei der weiteren und der noch weiteren Ausführungsform dadurch von seinem Katheter-Aufnahmeglied gelöst, dass das betreffende Katheter-Aufnahmeglied in sein zugehöriges Katheter-Löseglied eingezogen wurde. In diesem Zusammenhang sei jedoch angemerkt, dass eine Platziervorrichtung gemäß der Erfindung auch so aufgebaut sein kann, dass in dem schlauchförmig ausgebildeten Katheter-Aufnahmeglied ein schlauch- oder zylinderförmiges Katheter-Löseglied aufgenommen ist. Die Bewegungen eines solchen Katheter-Aufnahmegliedes und eines solchen Katheter-Lösegliedes können den Bewegungen der oben beschriebenen Katheter-Aufnahmegliedern und Katheter-Lösegliedern völlig entsprechen.

### Bezugszeichenliste

- 1: Platziervorrichtung
- 2: Seitenteil
- 3: Seitenteil
- 4: Drückteil
- 5: Drückteil
- 6: Führungsbahn
- 7: Führungsbahn
- 8: Lagerzapfen
- 9: Antriebselement
- 10: Katheter-Löseglied
- 11: Katheter-Aufnahmeglied
- 12: Tragteil
- 13: Führungsbahnabsenkung
- 13': Führungsbahnabsenkung
- 13": Führungsbahnabsenkung
- 14: Katheter
- 15: Verbindungsteil
- 16: Drückteil
- 17: Drückteil
- 18: Verbindungsteil
- 19: Tragarm
- 20: Tragarm
- 21: Öffnung
- 22: Öffnung
- 23: Gelenkglied
- 24: Gelenkglied
- 30: Halter
- 31: Betätigungsglied
- 32: Lagerzapfen
- 33: Führungsbahn
- 34: Schiebeglied
- 35: Längsloch
- 40: Aufnahmekörper
- 41: Schwenkglied, Hebel
- 42: Schiebeglied
- 43: Längsloch
- 44: Lageröffnung
- 45: Aufnahmeöffnung
- 46: Lagerzapfen
- 47: Stabilisierungsrippe
- 48: Sicherungsteil
- 49: Griffteil
- 50: Aufnahmeöffnung
- 51: Einsteckteil
- 52: Einsteckteil
- 53: Klemm- bzw. Rastnase
- 54: Anlagespitze
- 55: Anlagespitze
- 56: Detektierschicht

## Patentansprüche

1. Platziervorrichtung für einen in einer Körperöffnung oder -höhle eines Individuums zu positionierenden medizinischen Katheter (14), umfassend
- ein längliches rohr- oder schlauchförmiges Katheter-Aufnahmeglied (11), von dessen einen Ende der zu platzierende Katheter (14) aufnehmbar ist,
- ein innerhalb oder außerhalb des Katheter-Aufnahmegliedes (11) aufgenommenes und in dessen Längsrichtung verschiebbares längliches Katheter-Löseglied (10)
- und einen zwischen dem Katheter-Aufnahmeglied (11) und dem Katheter-Löseglied (10) vorgesehenen Bewegungsmechanismus mit einer Bewegungsumsetzeinrichtung (16, 17, 18, 19, 20), durch die das Katheter-Aufnahmeglied (11) mit dem von diesem aufgenommenen Katheter (14) und das Katheter-Löseglied (10) in einer Vorrichtungslängsrichtung relativ zueinander unter Loslösen des Katheters (14) von dem Katheter-Aufnahmeglied (11) verschiebbar sind,
- wobei die Bewegungsumsetzeinrichtung (16, 17, 18, 19, 20) ein Betätigungsglied (16, 17), durch das eine quer zu der genannten Vorrichtungslängsrichtung ausgeübte Transversalbewegung oder eine Schwenkbewegung in eine in der genannten Vorrichtungslängsrichtung verlaufende Longitudinalbewegung umsetzbar ist, und zwei Drückteile (16, 17) aufweist, die durch einen Verbindungsteil (15) miteinander verbunden und in Richtung zu diesem hin elastisch zusammendrückbar sind,
- wobei der Verbindungsteil (15) mit dem Katheter-Aufnahmeglied (11) oder dem Katheter-Löseglied (10) fest verbunden ist,
- und wobei das Katheter-Löseglied (10) oder das Katheter-Aufnahmeglied (11) mit den Drückteilen (16, 17) mittels Gelenkgliedern (18, 19, 20) gekoppelt ist, die aus zwei Tragarmen (19, 20) und einem diese verbindenden Verbindungsteil (18) bestehen, um den die Tragarme (19, 20) elastisch zusammendrückbar sind.

2. Platziervorrichtung nach Anspruch 1, **dadurch gekennzeichnet, dass** die Tragarme (19, 20) mit Koppelelementen (23, 24) der Drückteile (16, 17) gelenkig verbunden sind.

3. Platziervorrichtung nach Anspruch 1, **dadurch gekennzeichnet, dass** die Tragarme (19, 20) mit den Drückteilen (16, 17) zusammenhängend einteilig ausgebildet sind.

4. Platziervorrichtung nach einem der Ansprüche 1 bis 3,
**dadurch gekennzeichnet, dass** zwischen dem Katheter-Aufnahmeglied (11) und dem Katheter-Löseglied (10) eine strahlungsempfindliche Detektierschicht (52) vorgesehen ist.

## Claims

1. A positioning apparatus for a medical catheter (14) to be positioned in a body opening or cavity of an individual, comprising
- an elongated tubular or hose-shaped catheter accommodation member (11), by one end of which the catheter (14) to be positioned can be accommodated,
- an elongated catheter release member (10) accommodated within or outside of the catheter accommodation member (11) and displaceable in the longitudinal direction thereof,
- and a movement mechanism provided between the catheter accommodation member (11) and the catheter release member (10) having a movement conversion device (16, 17, 18, 19, 20), by which the catheter accommodation member (11) with the catheter (14) accommodated by it and the catheter release member (10) are displaceable relative to each other in an apparatus longitudinal direction with release of the catheter (14) from the catheter accommodation member (11),
- wherein the movement conversion device (16, 17, 18, 19, 20) comprises an actuating member (16, 17), by which a transversal movement exerted transverse to the said apparatus longitudinal direction or a pivoting movement can be converted into a longitudinal movement extending in the said apparatus longitudinal direction, and two pressing parts (16, 17), which are connected to each other by a connecting part (15) and can be elastically compressed towards it,
- wherein the connecting part (15) is fixedly connected to the catheter accommodation member (11) or the catheter release member (10),
- and wherein the catheter release member (10) or the catheter accommodation member (11) is coupled to the pressing parts (16, 17) by means of hinge members (18, 19, 20), which are composed of two support arms (19, 20) and a connecting part (18) connecting them, around which the support arms (19, 20) can be elastically compressed.

2. The positioning apparatus according to claim 1, **characterized in that** the support arms (19, 20) are connected to coupling elements (23, 24) of the pressing parts (16, 17) in articulated manner.

3. The positioning apparatus according to claim 1, **characterized in that** the support arms (19, 20) are integrally formed contiguous with the pressing parts (16, 17).

4. The positioning apparatus according to any one of claims 1 to 3, **characterized in that** a radiation-sensitive detection layer (52) is provided between the catheter accommodation member (11) and the catheter release member (10).

## Revendications

1. Appareil de positionnement pour un cathéter médical (14), à positionner dans une ouverture ou une cavité corporelle d'un individu, comprenant :
- un élément récepteur de cathéter longitudinal, en forme de tuyau ou de tube (11), à partir de l'extrémité duquel le cathéter (14) à positionner peut être logé,
- un élément de desserrage de cathéter longitudinal (10), logé à l'intérieur ou à l'extérieur de l'élément récepteur de cathéter (11) et pouvant être déplacé dans sa direction longitudinale et
- un mécanisme de déplacement, prévu entre l'élément récepteur de cathéter (11) et l'élément de desserrage de cathéter (10), avec un dispositif de réalisation de mouvement (16, 17, 18, 19, 20), par le biais duquel l'élément récepteur de cathéter (11) peut être déplacé avec le cathéter (14), logé par celui-ci et l'élément de desserrage de cathéter (10), dans une direction longitudinale de l'appareil l'un par rapport à l'autre, avec détachement du cathéter (14) de l'élément récepteur de cathéter (11),
- le dispositif de réalisation de mouvement (16, 17, 18, 19, 20) présentant un élément d'actionnement (16, 17), par le biais duquel un mouvement transversal, exercé transversalement par rapport à la direction longitudinale nommée de l'appareil ou un mouvement de pivotement dans un mouvement longitudinal, évoluant dans la direction longitudinale nommée de l'appareil, peut être réalisé et deux parties formant pression (16, 17), qui sont reliées l'une à l'autre par le biais d'une partie de raccordement (15) et qui peuvent être compressées élastiquement dans la direction de celle-ci,
- la partie de raccordement (15) étant reliée fixement à l'élément récepteur de cathéter (11) ou à l'élément de desserrage de cathéter (10) et
- l'élément de desserrage de cathéter (10) ou l'élément récepteur de cathéter (11) étant couplé avec les parties formant pression (16, 17) au moyen d'éléments d'articulation (18, 19, 20), qui se composent de deux bras porteurs (19, 20) et d'une partie de raccordement (18) qui les relie, autour de laquelle les bras porteurs (19, 20) sont compressibles élastiquement.

2. Appareil de positionnement selon la revendication 1,
**caractérisé en ce que**
les bras porteurs (19, 20) sont reliés de façon articulée à des éléments de couplage (24, 24) des éléments d'actionnement (16, 17).

3. Appareil de positionnement selon la revendication 1,
**caractérisé en ce que**
les bras porteurs (19, 20) sont constitués d'un seul tenant, de façon continue, avec les parties formant pression (16, 17).

4. Appareil de positionnement selon l'une des revendications 1 à 3,
**caractérisé en ce que**,
entre l'élément récepteur de cathéter (11) et l'élément de desserrage de cathéter (10), il est prévu une couche de détection (52), sensible aux rayonnements.
